(19) Europäisches Patentamt
European Patent Office
Office européen des brevets

(11) **EP 0 539 814 B1**

(12) **EUROPÄISCHE PATENTSCHRIFT**

(45) Veröffentlichungstag und Bekanntmachung des Hinweises auf die Patenterteilung:
**27.12.1996 Patentblatt 1996/52**

(51) Int Cl.6: **C12M 1/40**, C12Q 1/54, G01N 33/487, G01N 27/49

(21) Anmeldenummer: **92117755.6**

(22) Anmeldetag: **16.10.1992**

(54) **Elektrokatalytischer Glucosesensor**

Electrocatalytic glucose sensor

Capteur électrocatalytique de glucose

(84) Benannte Vertragsstaaten:
**DE FR GB IT SE**

(30) Priorität: **29.10.1991 DE 4135633**

(43) Veröffentlichungstag der Anmeldung:
**05.05.1993 Patentblatt 1993/18**

(73) Patentinhaber: **SIEMENS AKTIENGESELLSCHAFT**
**80333 München (DE)**

(72) Erfinder: **Tegeder, Volker, Dr.**
**W-8520 Erlangen (DE)**

(56) Entgegenhaltungen:
**EP-A- 0 101 880      EP-A- 0 103 109**
**DE-A- 2 936 652**

• **PATENT ABSTRACTS OF JAPAN vol. 13, no. 430 (P-937) 26. September 1989 & JP-A-01 163 649 (SONY CORP) 27. Juni 1989**

**Beschreibung**

Die Erfindung betrifft einen elektrokatalytischen Sensor zur Bestimmung von Glucose, insbesondere in Körperflüssigkeiten.

Die Messung der Glucosekonzentration in Körperflüssigkeiten, insbesondere im Blut eines Patienten, ist beispielsweise bei der Diabetestherapie erforderlich. Dabei soll nämlich - mit Hilfe einer elektronisch gesteuerten Pumpe - eine an den aktuellen Bedarf angepaßte automatische Insulindosierung erfolgen. Dazu wird ein implantierbarer Sensor benötigt, der spezifisch auf Glucose anspricht, eine gute Langzeitstabilität aufweist und biokompatibel ist. Ein derartiger Glucosesensor wäre zudem auch bei klinischen Untersuchungen für die Glucosemessung und bei Glucosetoleranztests einsetzbar, d.h. er muß nicht notwendigerweise implantiert werden und die Glucosebestimmung kann beispielsweise im Serum erfolgen.

Die Bestimmung von Glucose in Körperflüssigkeiten kann mit Enzymsensoren und mit elektrokatalytischen Sensoren erfolgen. Enzymsensoren, bei denen im allgemeinen Glucoseoxidase als Enzym verwendet wird, weisen den Nachteil einer mangelnden Langzeitstabilität auf. Elektrokatalytische Glucosesensoren, bei denen die elektrochemische Oxidation von Glucose als Meßsignal dient, werden durch andere Bestandteile der Körperflüssigkeit, die ebenfalls elektrochemisch oxidierbar sind, gestört. Deshalb ist die Meßgenauigkeit auf ca. 10 bis 20 % begrenzt. Elektrokatalytische Glucosesensoren weisen im übrigen im allgemeinen eine Meß- bzw. Arbeitselektrode, eine Bezugselektrode und eine Gegenelektrode auf, wobei vor der aktiven Fläche der Meßelektrode eine hydrophile Membran angeordnet ist (siehe dazu beispielsweise EP-PS 0 103 109).

Aufgabe der Erfindung ist es, einen elektrokatalytischen Glucosesensor der eingangs genannten Art in der Weise auszugestalten, daß die Selektivität bzw. die Empfindlichkeit erhöht wird und somit eine Verbesserung der Meßgenauigkeit erreicht werden kann.

Dies wird erfindungsgemäß durch einen Sensor erreicht, der folgende Merkmale aufweist:

- eine Reaktionselektrode zur Umsetzung von Glucose zu Glucono-δ-lacton,
- eine im geringen Abstand hinter der Reaktionselektrode angeordnete Arbeitselektrode zum Nachweis von Glucono-δ-lacton oder Glucose,
- eine vor der Reaktionselektrode auf der von der Arbeitselektrode abgewandten Seite angeordnete hydrophile Membran,
- eine Bezugselektrode und
- eine Gegenelektrode.

Beim Glucosesensor nach der Erfindung ist vor der Arbeitselektrode, in geringem Abstand davon, eine weitere Elektrode angeordnet, die sogenannte Reaktionselektrode. An dieser Elektrode wird, vorzugsweise bei niedrigem Potential, Glucose elektrokatalytisch zu Glucono-δ-lacton umgesetzt und angereichert. Dieses Lacton wird dann an der Arbeitselektrode elektrochemisch oxidiert, d.h. über den Elektrooxidationsstrom nachgewiesen. Die dabei resultierende Ladung ergibt ein von der Glucosekonzentration abhängiges Meßsignal. Auf diese Weise gelingt es, die Selektivität des Glucosesensors zu verbessern und den Meßfehler auf unterhalb 10 % zu senken.

Beim erfindungsgemäßen elektrokatalytischen Glucosesensor werden - zur Erhöhung der Spezifität - besondere kinetische Eigenschaften der Glucoseelektrooxidation ausgenutzt. Bei der Elektroadsorption von Glucose an einer Platinelektrode wird als Reaktionsprodukt nämlich Glucono-δ-lacton gebildet (siehe dazu: "Ber. Bunsenges. Phys. Chem.", Bd. 84 (1980), Seiten 50 bis 55, sowie "J. Electroanal. Chem.", Vol. 284 (1990), Seiten 335 bis 349). Dieses Lacton, das auch als Gluconsäure-δ-lacton bzw. Gluconsäure-5-lacton bezeichnet wird, wird mit einer Halbwertszeit von ca. 10 min zu Gluconsäure hydrolysiert (siehe dazu: "Acta Chem. Scand.", Vol. A 36 (1982), Seiten 417 bis 422). Ein Nachweis von Glucono-δ-lacton kann deshalb als besonders spezifisch für den Glucosenachweis angesehen werden, da es nur durch die Elektroadsorption der Glucose direkt an der Elektrode entsteht. Der erfindungsgemäße Glucosesensor ermöglicht somit einen besonders empfindlichen und spezifischen Nachweis von Glucose, und zwar sowohl einen direkten als auch einen indirekten Nachweis.

Der Glucosesensor nach der Erfindung weist ein Elektrodensystem aus einer Reaktionselektrode und einer - in geringem Abstand davon angeordneten - Arbeitselektrode auf. Diese Anordnung wird in der Weise realisiert, daß zwischen Reaktionselektrode und Arbeitselektrode ein Abstandhalter vorgesehen ist. Dies kann beispielsweise eine beliebige poröse Struktur sein. Vorteilhaft ist zu diesem Zweck jedoch zwischen Reaktionselektrode und Arbeitselektrode eine Separatormembran mit einem hohen Diffusionskoeffizienten für Glucono-δ-lacton angeordnet. Als Separator dient deshalb beispielsweise eine 10 μm dicke Membran mit einem Diffusionskoeffizienten (für das Lacton) $D > 10^{-7}$ $cm^2/s$. Reaktionselektrode und Separatormembran können auch eine Baueinheit bilden.

Zur Körperflüssigkeit bzw. zum Elektrolyten hin wird die Elektrodenanordnung durch eine äußere Membran begrenzt, d.h. abgedeckt. Diese Membran, die hydrophil ist und im Falle einer Implantation des Sensors aus biokompatiblem Material bestehen muß, hat die Aufgabe, größere Moleküle als Glucose von den Elektroden fernzuhalten. Um den Lacton-Diffusionsstrom zur Arbeitselektrode zu lenken, ist der Diffusionskoeffizient der Separatormembran deutlich größer als derjenige der äußeren, vor bzw. auf der Reaktionselektrode angeordneten Membran. Bei einer extrakorporalen Anwendung des Sensors kann die hydrophile Membran vor der Reaktionselektrode auch entfallen.

Die Reaktionselektrode weist vorteilhaft eine große wirksame Oberfläche auf, wodurch ermöglicht wird, daß die Meßzeit kurz ist. Vorzugsweise dient als Reaktionselektrode ein feinmaschiges Platinnetz, es können beispielsweise aber auch großflächige, poröse Elektroden aus einer Platin/Iridium- oder Platin/ Gold-Legierung verwendet werden. Die Arbeitselektrode besteht vorzugsweise aus Platin, daneben können beispielsweise auch Platin/Iridium- und Platin/Gold-Legierungen eingesetzt werden. Die Arbeitselektrode ist dabei vorteilhaft oberflächlich aufgerauht.

Der Glucosesensor nach der Erfindung kann vorteilhaft als katheterförmiger Sensor ausgebildet sein. In einen derartigen Sensor sind dann sowohl eine Gegen- und eine Bezugselektrode als auch ein Elektrodensystem der vorstehend genannten Art - mit Arbeits- und Reaktionselektrode sowie Membran(en) - integriert. Die Gegenelektrode und die Bezugselektrode, die im übrigen auch eine Baueinheit bilden können, sowie das Elektrodensystem sind dabei auf dem Kathetersensor - mit Abstand voneinander - hintereinander angeordnet.

Beim erfindungsgemäßen elektrokatalytischen Sensor wird, wie bereits ausgeführt, das an der Reaktionselektrode gebildete Glucono-δ-lacton an der Arbeitselektrode nachgewiesen. Dies geschieht vorteilhaft nach dem sogenannten Potentialsprungverfahren (siehe dazu EP-PS 0 103 109). Dabei erfolgt vorzugsweise eine Differenzmessung, d.h. es wird mit und ohne angelegtem Potential an der Reaktionselektrode gemessen. Durch eine derartige Betriebsweise wird eine hohe Spezifität erreicht.

Das Potential der Reaktionselektrode (Reaktionspotential) wird so niedrig gewählt, d.h. 0 bis 350 mV, beispielsweise 130 mV, bezogen auf die reversible Wasserstoffelektrode, daß möglichst nur Glucose umgesetzt wird. Die bei dieser Umsetzung gebildeten Adsorbate werden dann periodisch durch Anlegen eines hohen Potentials (Reaktivierungspotential) entfernt, beispielsweise bei 1630 mV, bezogen auf die reversible Wasserstoffelektrode; allgemein beträgt dieses Potential $\geq$ 1400 mV, insbesondere 1400 bis 1900 mV. Die an der Reaktionselektrode auftretenden Ströme (Adsorptions- bzw. Reaktivierungsstrom) können - zur Bestimmung der Glucosekonzentration - zusätzlich gemessen und in geeigneter Weise in die Auswertung des Meßergebnisses an der Arbeitselektrode mit einbezogen werden.

Wird der erfindungsgemäße Glucosesensor nach dem Potentialsprungverfahren betrieben, so erfolgt die Potentialbelastung der Arbeitselektrode vorteilhaft in der Weise, daß dieser Elektrode nacheinander drei unterschiedliche Potentiale aufgeprägt werden. Bei einem dieser Potentiale, dem Meßpotential, wird der Strom integriert, um den Wert für die Ladung zu erhalten. Die beiden anderen Potentiale sind ein Vorpotential (Reduktionspotential) und ein Reaktivierungspotential (Oxidationspotential). Die drei Potentiale liegen beispielsweise bei folgenden Werten, jeweils bezogen auf die reversible Wasserstoffelektrode (RHE):

- Vorpotential: 0 bis 350 mV, beispielsweise 130 mV;
- Meßpotential: 600 bis 850 mV, beispielsweise 750 mV;
- Reaktivierungspotential: $\geq$ 1400 mV, insbesondere 1400 bis 1900 mV, beispielsweise 1630 mV.

Die Elektrodenpotentiale nehmen folgenden zeitlichen Verlauf. Die Potentiale (der beiden Elektroden) können sowohl gleichzeitig als auch sukzessive an die jeweilige Elektrode angelegt werden, und zwar unter Verwendung eines Bipotentiostaten bzw. eines normalen Potentiostaten. Bei der Verwendung eines normalen Potentiostaten wird zunächst die Reaktionselektrode belastet. Dabei wird an die Reaktionselektrode zuerst das Reaktionspotential angelegt, und zwar für 5 s bis 5 min, beispielsweise für 10 s, und dann das Reaktivierungspotential, und zwar für 0,1 bis 10 s, beispielsweise für 1 s. Durch periodische Wiederholung dieser Belastung, beispielsweise 10 mal, reichert sich dann, begrenzt durch den Glucosestrom, die Diffusion in den Elektrolyten und die Hydrolyse (des Lactons), das Glucono-δ-lacton vor der Arbeitselektrode an.

Anschließend erfolgt die Belastung der Arbeitselektrode, und zwar mit dem zyklischen Durchlaufen von Vor-, Meß- und Reaktivierungspotential. Die Belastungsdauer beträgt jeweils 0,1 bis 10 s beim Vor- und beim Reaktivierungspotential, beispielsweise jeweils 1 s, und 1 bis 100 s, beispielsweise 10 s, beim Meßpotential. Die Arbeitselektrode wird dabei so lange belastet, beispielsweise ca. 2 min, bis die gemessene Ladung Q, abhängig vom Lacton-Diffusionskoeffizienten der äußeren Membran, deutlich abgefallen ist. Aus der Differenz der anfänglich, beispielsweise bei den ersten zehn Zyklen (gemittelt), gemessenen Ladung $Q_1$ beim Meßpotential und der später, beispielsweise bei den letzten zehn Zyklen (gemittelt), gemessenen Ladung $Q_2$ ergibt sich dann - über eine Kalibrierung - das spezifische Sensorsignal.

Durch die Differenzmessung wird die Selektivität erhöht, und zwar aus folgenden Gründen. Die gemessene Ladung $Q_i$ resultiert aus den Oxidationsströmen des Lactons $Q_i^L$, der Glucose $Q_i^G$ und der Störsubstanzen $Q_i^S$. Daraus ergibt sich für das Signal $\Delta Q$ folgendes: $Q = Q_1 - Q_2 = (Q_1^L + Q_1^G + Q_1^S) - (Q_2^L + Q_2^G + Q_2^S)$. Wenn nun das Reaktionspotential möglichst niedrig gewählt wird, so daß an der Reaktionselektrode keine Störsubstanzen umgesetzt werden, dann gilt: $Q_1^S = Q_2^S$. Außerdem setzt eine möglichst großflächige Reaktionselektrode die Glucose weitestgehend in Glucono-δ-lacton um, so daß $Q_1^G \approx 0$ gesetzt werden kann. Ferner wird die Messung von $Q_2$ erst durchgeführt, wenn $Q_2^L \sim 0$ ist. Daraus folgt: $Q = Q_1^L - Q_2^G$.

Das Signal $\Delta Q$ erreicht somit dann seinen maximalen Wert, wenn durch eine großflächige Reaktionselektrode möglichst viel Lacton angereichert wird, womit $Q_1^L$ groß wird, und wenn - bedingt durch die geringere Re-

aktivität von Glucose - $Q_2^G$ möglichst klein ist. Bei der geschilderten Vorgehensweise ergibt sich die gewünschte Erhöhung der Selektivität somit daraus, daß Änderungen in der Konzentration der Störsubstanzen, für die $Q_1^S = Q_2^S$ gilt, keinen Einfluß auf das Meßsignal $\Delta Q$ haben.

Das spezielle Elektrodensystem des erfindungsgemäßen Glucosesensors, das eine Erhöhung der Konzentration von Glucono-δ-lacton vor der Arbeitselektrode bewirkt, ergibt auch bei der Verwendung anderer Meßverfahren eine höhere Empfindlichkeit _ und/oder Spezifität, da eine Differenzmessung mit und ohne angelegtem Reaktionspotential möglich ist. Ein derartiges weiteres Meßverfahren ist insbesondere das sogenannte Impedanzverfahren (siehe dazu EP-PS 0 101 880). Bei diesem Verfahren wird der Arbeitselektrode ein innerhalb eines Potentialintervalls zeitlich in Potentialstufen variierendes Potential aufgeprägt und diesem Potential eine Wechselspannung vorgegebener Amplitude und Frequenz überlagert, wobei wenigstens für eine Potentialstufe der Real- und/oder der Imaginär anteil der Impedanz (der Arbeitselektrode) ermittelt und daraus die Konzentration bestimmt wird.

## Patentansprüche

1. Elektrokatalytischer Sensor zur Bestimmung von Glucose, insbesondere in Körperflüssigkeiten, **gekennzeichnet durch**:

    - eine Reaktionselektrode zur Umsetzung von Glucose zu Glucono-δ-lacton,
    - eine im geringen Abstand hinter der Reaktionselektrode angeordnete Arbeitselektrode zum Nachweis von Glucono-δ-lacton oder Glucose,
    - eine vor der Reaktionselektrode auf der von der Arbeitselektrode abgewandten Seite angeordnete hydrophile Membran,
    - eine Bezugselektrode und
    - eine Gegenelektrode.

2. Sensor nach Anspruch 1, **dadurch gekennzeichnet,** daß zwischen Reaktionselektrode und Arbeitselektrode eine Separatormembran mit einem hohen Diffusionskoeffizienten für Glucono-δ-lacton angeordnet ist.

3. Sensor nach Anspruch 1 oder 2, **dadurch gekennzeichnet,** daß die Reaktionselektrode eine große wirksame Oberfläche aufweist.

4. Sensor nach Anspruch 3, **dadurch gekennzeichnet,** daß die Reaktionselektrode ein feinmaschiges Platinnetz ist.

5. Sensor nach einem der Ansprüche 1 bis 4, **dadurch gekennzeichnet,** daß die Arbeitselektrode aus Platin besteht.

6. Sensor nach einem oder mehreren der Ansprüche 1 bis 5, **dadurch gekennzeichnet,** daß die Arbeitselektrode oberflächlich aufgerauht ist.

7. Sensor nach einem oder mehreren der Ansprüche 1 bis 6, **dadurch gekennzeichnet,** daß er katheterförmig ausgebildet ist.

8. Sensor nach einem oder mehreren der Ansprüche 1 bis 7, **dadurch gekennzeichnet,** daß die Umsetzung der Glucose an der Reaktionselektrode bei einem Potential von 0 bis 350 mV erfolgt, bezogen auf die reversible Wasserstoffelektrode.

## Claims

1. Electrocatalytic sensor for determining glucose, particularly in body fluids, characterised by:

    - a reaction electrode for converting glucose into glucono-δ-lactone,
    - a working electrode which is arranged at a slight distance behind the reaction electrode, for detecting glucono-δ-lactone or glucose,
    - a hydrophilic membrane arranged in front of the reaction electrode on the side which faces away from the working electrode,
    - a reference electrode and
    - a counter electrode.

2. Sensor according to claim 1, characterised in that arranged between the reaction electrode and the working electrode is a separator membrane having a high diffusion coefficient for glucono-δ-lactone.

3. Sensor according to claim 1 or 2, characterised in that the reaction electrode has a large active surface.

4. Sensor according to claim 3, characterised in that the reaction electrode is a fine-mesh platinum net.

5. Sensor according to one of the claims 1 to 4, characterised in that the working electrode consists of platinum.

6. Sensor according to one or more of the claims 1 to 5, characterised in that the working electrode is roughened on the surface.

7. Sensor according to one or more of the claims 1 to 6, characterised in that it is constructed in a manner such that it is catheter-shaped.

8. Sensor according to one or more of the claims 1 to 7, characterised in that the conversion of the glucose at the reaction electrode takes place at a potential of 0 to 350 mV, with respect to the reversible hydrogen electrode.

## Revendications

1. Capteur électrocatalytique pour la détermination du glucose, en particulier dans des liquides du corps, caractérisé par :

   - une électrode de réaction pour la transformation du glucose en δ-lactone de l'acide gluconique,
   - une électrode de travail disposée à faible distance après l'électrode de réaction, pour identifier le δ-lactone de l'acide gluconique ou le glucose,
   - une membrane hydrophile disposée avant l'électrode de réaction, sur le côté détourné de l'électrode de travail,
   - une électrode de référence et
   - une contre-électrode.

2. Capteur selon la revendication 1, caractérisé en ce qu'une membrane de séparation à fort coefficient de diffusion pour le δ-lactone de l'acide gluconique est disposée entre l'électrode de réaction et l'électrode de travail.

3. Capteur selon l'une des revendications 1 ou 2, caractérisé en ce que l'électrode de réaction a une surface effective importante.

4. Capteur selon la revendication 3, caractérisé en ce que l'électrode de réaction est une grille en platine à mailles fines.

5. Capteur selon l'une des revendications 1 à 4, caractérisé en ce que l'électrode de travail est en platine.

6. Capteur selon l'une ou plusieurs des revendications 1 à 5, caractérisé en ce que l'électrode de travail a une surface rugueuse.

7. Capteur selon l'une ou plusieurs des revendications 1 à 6, caractérisé en ce qu'il est réalisé en forme de cathéter.

8. Capteur selon l'une ou plusieurs des revendications 1 à 7, caractérisé en ce que la transformation du glucose à l'électrode de réaction s'effectue à un potentiel compris entre 0 et 350 mV, par rapport à l'électrode à hydrogène réversible.